# EUROPEAN PATENT SPECIFICATION

(11) **EP 1 984 077 B1**
(45) Date of publication and mention of the grant of the patent: **26.09.2012**
(21) Application number: 07705735.4
(22) Date of filing: 30.01.2007
(51) Int. Cl.: A61N 5/06

(54) **Body cover, glasses and/or at least partial head cover for radiating at least part of a human body**
Körperabdeckung, Gläser und/oder mindestens teilweise Kopfabdeckung zur Bestrahlung mindestens eines Teils des menschlichen Körpers
Elément couvrant le corps, lunettes et/ou élément couvrant au moins partiellement la tête permettant d'effectuer une irradiation au moins partielle d'un corps humain

(30) Priority: 06.02.2006 EP 06003887
(43) Date of publication of application: 29.10.2008
(73) Proprietor: Koninklijke Philips Electronics N.V., 5621 BA Eindhoven (NL)
(72) Inventor: ASVADI, Sima, NL-5656 AA Eindhoven (NL); HENDRIKS, Bernardus, H., W., NL-5656 AA Eindhoven (NL); ENGELS, Philomeen, A., C., NL-5656 AA Eindhoven (NL); VAN DEN BELD, Gerrit, J., NL-5656 AA Eindhoven (NL); DE GRAAF, Jan, NL-5656 AA Eindhoven (NL); VAN HOUTEN, Henk, NL-5656 AA Eindhoven (NL)
(74) Representative: Uittenbogaard, Frank
(86) International application number: PCT/IB2007/050305
(87) International publication number: WO 2007/091188

(56) References cited:
- US-A- 6 045 575
- US-A1- 2003 167 080
- US-A1- 2004 138 726
- US-A1- 2005 085 875
- US-B1- 6 596 016
- US-B1- 6 858 036

## Description

### FIELD OF THE INVENTION

The invention relates in general to a body cover, comprising a substrate, for covering at least part of a human body, and at least one electroluminescent source coupled to the substrate.

### BACKGROUND OF THE INVENTION

Light is known to have beneficial beneficial effects on humans. In fields such as phototherapy light can be used for healing purposes. A case where light treatment is used is hyperbilirubinemia. Hyperbilirubinemia patients are daily exposed to a high dose of blue light for a period up to eleven hours. Hyperbilirubinemia is a disorder of bilirubin metabolism. Bilirubin is the toxic waste product of the worn-out red blood cells that get converted by an enzyme produced by the liver, which excretes the bilirubin. Hyperbilirubinemia patients lack this enzyme, which causes an accumulation of the bilirubin in the tissue. This results in jaundice of the skin and the eyes. Neonatal jaundice and crigler najjar are two types of Hyperbilirubinemia. Crigler najjar patients range from the age of infant to adult and need daily light therapy for life, for various periods as long as 11 hours a day. Neonatal patients oftentimes need continuous light therapy for a few days.

Bilirubin degrades under influence of phototherapy. The therapeutic efficacy of phototherapy is, among others, dependent on the spectral light qualities, the intensity of the light, the exposed body surface area and the duration of exposure. A high-intensity narrow band blue light is used with a wavelength in the 450-470 nm spectrum, centered around a 450 nm peak and no light emission in the UV wavelength area. Light intensities of at least 5mW/cm² up to 10 mW/cm² and full body exposure are used for keeping treatment durations relatively acceptible.

Known treatments for jaundice, particularly baby jaundice, combine a warming box with light treatment. The warming box is required since the baby has to be naked for the efficiency of the light treatment, making the normal process of taking care of the baby, for example breast feeding, more difficult and limiting its freedom of movement. The eyes of the baby are covered to be protected against bright light.

Known phototherapy devices for treatment of hyperbilirubinemia commonly use fluorescent tubes or halogen spots. Disadvantages of these devices include physical size and shape, heat production, unstable and broad wavelength light output, limited body surface area illumination and UV radiation. The conventional phototherapy devices illuminate the patients from one side, for example, because it is uncomfortable to lie on a transparant plastic sheet or transparant fabric over the hot lamps for a long period. In some cases a transparent bed provides the opportunity for exposing the body from both sides, however, this bed doesn't meet the requirements of a comfortable bed.

Furthermore, a small fibreoptic sheet to illuminate a baby is developed. Drawbacks are the limited size and relative low intensity. Hence it is commonly used in combination with other phototherapy devices and besides, it can not be used for older patients.

Furthermore it is said that light having a wavelength of approximately 450 nm emitted to human eyes has a revitalising effect on a person. It is also known that phototherapy is used for stimulating purposes of cells in/near the skin, for example in wound healing, at different wavelengths within a wide range up to the infrared region.

US 6,596,016 discloses a phototherapy garment which contains a flexible backing material, a transparent liner, and a flexible printed circuit sheet containing surface-mounted light-emitting diodes positioned between the backing material. The garment may contain a feedback system with skin bilirubin sensors so that the intensity level and duration of the light therapy can be based on the bilirubin in the skin.

US 2004/0138726 discloses a device for delivering light to the blood supply of a human body through a non-ocular skin area. The device has a plurality of light emitting diodes spaced apart from each other and extending through apparatuses for emitting light to the body. The device provides wavelengths of light within a specifically-determined range of intensities and a specifically determined angels of illumination. This document discloses the preamble of claim 1.

US 6,045,575 discloses an apparatus for treating neonatal jaundice. The garment has semiconductor light sources affixed thereto for radiating toward the "inside" of the garment when the infant is dressed in the garment.

US6,858,036 discloses a device for acupuncture comprising a pressure plate. The pressure plate is provided with a plurality of fitting rods and when it is desired to assemble two or more pressure plates into an assembly the fitting rods of one plate are elastically coupled to the channelled fitting tubes of another plate.

US2005/085875 discloses a system and method of administering photon therapy. The system includes a plurality of treatment modules and a case for housing the photon emitter. The case includes linkers for linking each of the treatment modules to form an arbitrary modular pattern.

### SUMMARY OF THE INVENTION

An object of the invention is to provide a relatively comfortable way of applying phototherapy.

This object and other objects are achieved by a body cover according to claim 1.

Said object and other objects are also achieved by a body cover according to the dependent claims.

### BRIEF DESCRIPTION OF THE DRAWINGS

Fig. 1 shows a diagram of a cover;
Fig. 2 schematically shows a top view foil with LEDs;
Fig. 3 schematically shows a perspective view of an interwoven yarn structure;
Figs. 4, 5 and 6 show a cover;
Figs. 7 and 8 show a cover as a sleeping bag;
Fig. 9 shows a cover as a sleeping bag for babies;
Fig. 10 shows a cover as a sleeping bag for infants;
Fig. 11 shows a modular cover for different sizes;
Fig. 12 shows a cover as a suit;
Fig. 13 shows a cover as a suit with a hood;
Fig. 14 shows a cover with an air cooling system;
Fig. 15 shows a detail of the air cooling system;
Fig. 16 shows a cover with a liquid cooling system;
Fig. 17 shows a mattress with a cover;
Fig. 18 shows a cover with arcs;
Fig. 19 shows a cover as a visor with LEDs;
Fig. 20 shows a cover as glasses with LEDs;
Fig. 21 shows a cover as a bra with LEDs;
Fig. 22 shows a cover as a lining for a bra with LEDs;
Fig. 23 shows a cover as a bra with LEDs.

In this description, identical or corresponding parts have identical or corresponding reference numerals. The exemplary embodiments shown should not be construed to be limitative in any manner and serve merely as illustration.

### DETAILED DESCRIPTION OF THE EMBODIMENTS

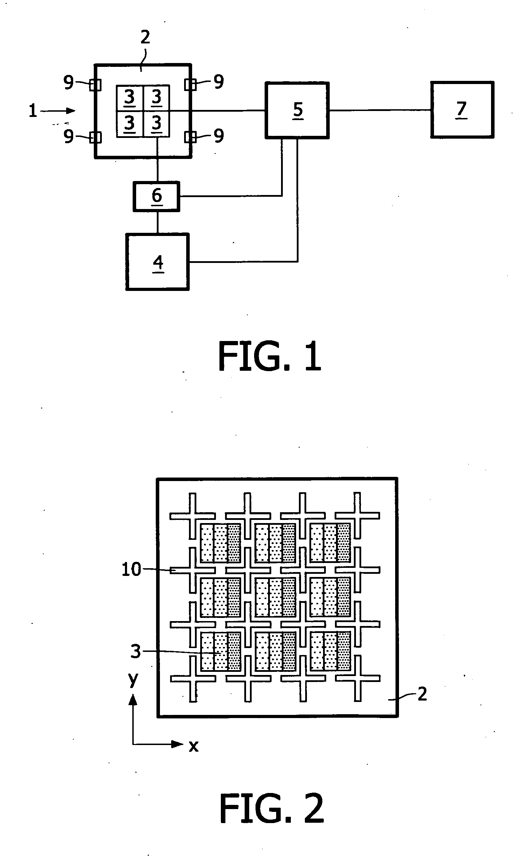

A cover 1 with electroluminescent sources for illuminating at least part of the body is shown in figure 1. The cover 1 comprises a substrate 2 which has electroluminescent sources 3 attached. The electroluminescent sources 3 are individually driven by power supply 4, which is controlled by control means 5. To individually address the electroluminescent sources 3, the power supply 4 may comprise a driver unit. Wavelength adjusting means 6 are provided for the possibility of varying the wavelength of at least a part of the electroluminescent sources 3 and are controlled by control means 5. By means of a user interface 7 the control means 5 can be set and/or adjusted to specify the settings of the electroluminescent sources 3. For instance by signalling the wavelength adjusting means 6, the wavelength of the electroluminescent sources 3 can be individually controlled, and/or it can be controlled in groups and/or all the sources 3 can be controlled. Also, by signalling the driver unit, the light output of the sources 3 can be controlled and/or turned on and off, for example.

The wavelength can for example be varied by turning on and off different electroluminescent sources 3 with different wavelengths. It is also possible to apply wavelength filters. By selecting the proper filters the proper wavelength can be selected. It is also possible to change the wavelength by varying the temperature of LEDs 3. Other wavelength adjusting means include using phosphors, preferably in combination with PWM (pulse width modulation) or varying the forward current, also preferably in combination with PWM. PWM can for example be applied to make sure that the intensity stays as desired while the wavelength is adjusted. Furthermore, the intensity of the light of the electroluminescent sources 3 can for example be set by means of adjusting the current through the power supply 4 for varying the light output. Of course, there are more ways of varying intensity, such as, by using PWM and/or by turning an amount of the electroluminescent sources 3 on/off, etc. The electroluminescent sources 3 are configured to emit therapeutic light to a part of a human body. Therefore, the cover 1 may for example be wrapped around a part of a human body, and/or around a body support such as a pillow, bed, chair, etc. The cover 1 is flexible and may be folded so that it is portable. Joining means 9 that join together are provided so that the cover 1 can be configured in a bag or cylinder shape and the part of the body can be surrounded by light from the electroluminescent sources 3. Joining means 9 may for example comprise, buttons, zippers, velcro, elements that can be tied together, elastic means for closing around at least a part of a body and/or body support, etc.

In an embodiment of the cover 1, the electroluminescent sources 3 comprise light emitting sources like LEDs (Light Emitting Diodes) 3, for example gallium-nitride blue LEDs. The latter are relatively effective, safe and convenient phototherapy devices and capable of delivering high intensity narrow wavelength band light. Furthermore, they can be configured into flexible devices, such as cover 1. The cover 1 is provided with a substrate 2 that is made of a flexible foil 2, in this case with LEDs 3 attached to it. Examples of such a flexible foil 2 provided with LEDs can be found in WO 2006/129272. An embodiment of the foil 2, at least, a part of a foil 2, with LEDs 3, is shown in figure 2. As can be seen from figure 2 the foil 2 is provided with rows and columns of openings 10, between the LEDs 3. The foil 2 can be constructed in different sizes. The openings 10 of the foil 2 increase flexibility and the breathing properties of the cover 1. Different embodiments of the foil 2 for example comprise knitted, woven and non-woven textiles and/or polymers, rubbery materials, spacer fabrics, foams,etc. The foil 2 is provided with not shown conducting lines of an electrical circuit, between the openings 10, for supplying power to and/or controlling the LEDs 3.

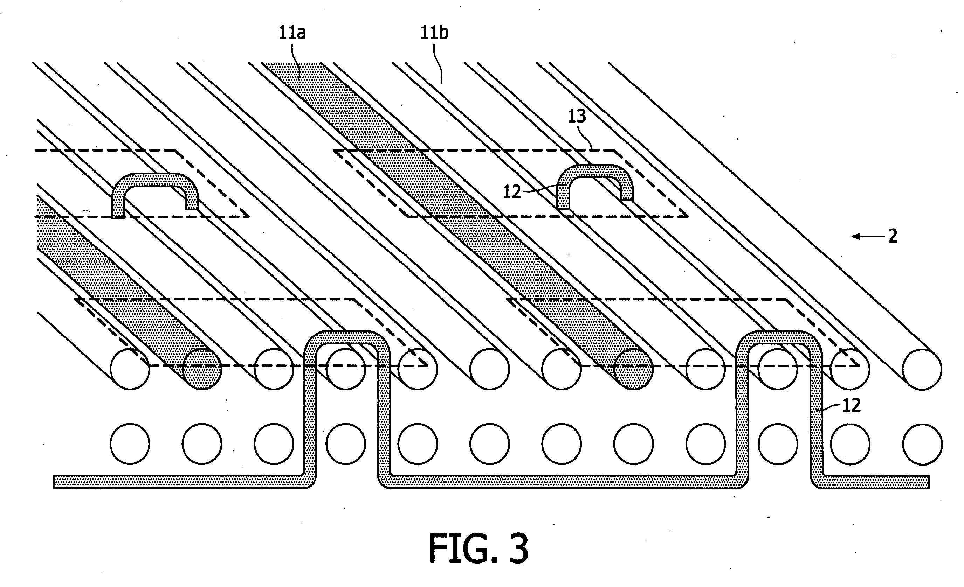

Another embodiment of the cover 1 is provided with LEDs 3 that are attached to yarns 11, 12 in a woven structure 2 of conducting and non-conducting yarns 11, 12. Such an embodiment can be found in WO 2006/129272. Figure 3 shows such an embodiment wherein the substrate 2 comprises a woven structure 2 of yarns 11, 12. The woven structure 2 is provided with conducting yarns 11a, 12 which comprise anodes 11a and cathodes 12. LEDs (not shown in figure 3) are connected with these conducting yarns 11, 12 at locations 13. In an embodiment, the configuration of the structure 2, more particularly, the yarns 11, 12, is such that the LEDs can be individually addressed. The structure 2 can comfortably cover a part of the body and illuminate it at the same time. The structure 2 can for example be a textile and can be combined with other (transparent) layers of textile, foil, polymers, etc. for example for extra protection of the body. Of course, the woven structure 2 doesn't have to be a textile but may also comprise a polymer such as nylon and/or polyester, etc. The structure 2 of this embodiment is flexible and can have the properties of body covers like clothes.

In another embodiment the electroluminescent sources 3 comprises OLEDs (organic LEDs) 3. OLEDs 3 are electroluminescent sources that can be configured to be flexible and/or transparent. Also, one OLED 3 can cover a large area of the body without being interrupted, while maintaining flexibility, so that a diffusing layer is not necessary, as opposed to many LEDs. The OLED 3 covering said large area can be driven by just one set of electrodes. The cover 1 can be provided with one or multiple OLEDs 3. OLEDs 3 are known in the art, and are for example described in WO 2007/054855.

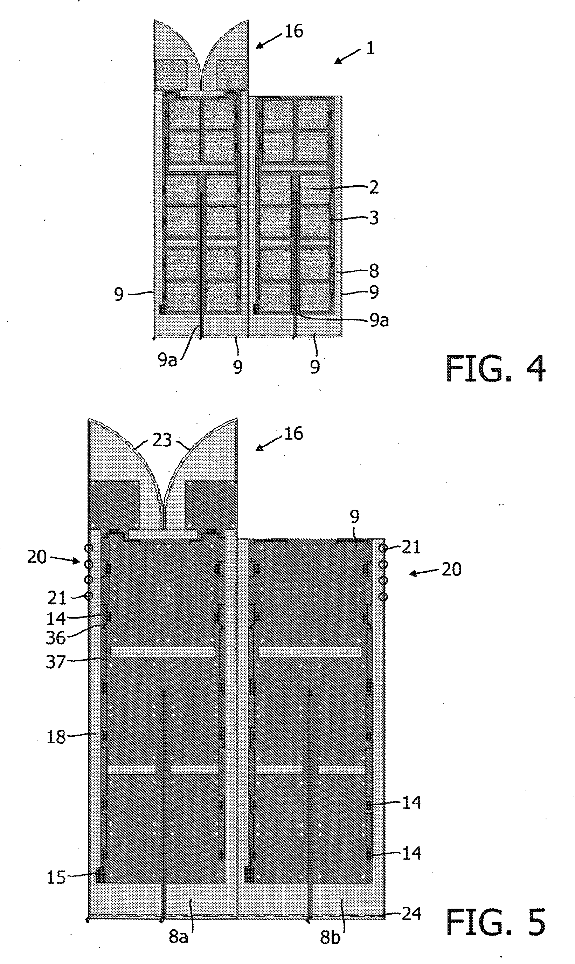

A cover 1 not part of the present invention is shown in figure 4. This cover comprises a flexible sheet 8 with foils 2 attached on the inside, for example, but not limited to foils 2 such as the foil 2 with LEDs 3 that is shown in figure 2. LEDs 3 (not shown in figure 2) are attached to the foil 2. A zipper 9 is provided along the edges of the sheet 8, so that the sheet 8 can be formed into a bag, for example a kind of sleeping bag. For example, the sheet 8 is made of a breathable material and contains strips of net material to enhance ventilation. The edges 18 of the sheet 8 may be made of a very breathable gauze material. Also pants forming zippers 9a may be provided, adding a hybrid function to the cover. Figure 5 shows such a cover 1, where the foils 2 with LEDs 3 are taken off. The foils 2 are attached to the sheet 8 by means of attachment means such as buttons 9, or for example velcro, magnetic means, static electricity, sticking means, etc. so that they are exchangeable, for example for washing or changing a defect foil 2 or for washing the cover 1 without damaging the LEDs 3 and/or the electrical ciruit of the foil 2. When attached, the foils 2, more particularly, the LEDs 3, connect with the foil connectors 14 that are provided at the location of where the foils 2 will be attached, at least approximately. The connectors 14 can also be integrated with said attachment means 9. The foil connectors 14 are connected to a plug contact 15, through which a connection is made with power supply 4 and in a particular embodiment, with control means 5. The foil connectors 14 are for example connected to the contact 15 by means of conducting lines or a cable 36. The cable 36 may be kept in place by straps 37 in the cover 1 and can easily be removed from the cover 1. In this way, the individual foils 2, more particularly the LEDs 3, can be driven and controlled by making simple contact with the foil connectors 14. For illumination around, or on the head or shoulders, flaps 16 are fixed to the cover 1.

The power supply 4 may comprise a battery 4, for example, but not limited to, a lithylene battery 4, which an be relatively flat shaped and preferably, relatively flexible. The cover may also be provided with connecting means for the electricity network, for example a plug. A not shown battery holder is provided for holding at least one battery 4. For continuous use of the cover 1 it is advantageous if batteries 4 can be exchanged without interfering with the functioning of the cover 1, particularly the electroluminescent sources 3.

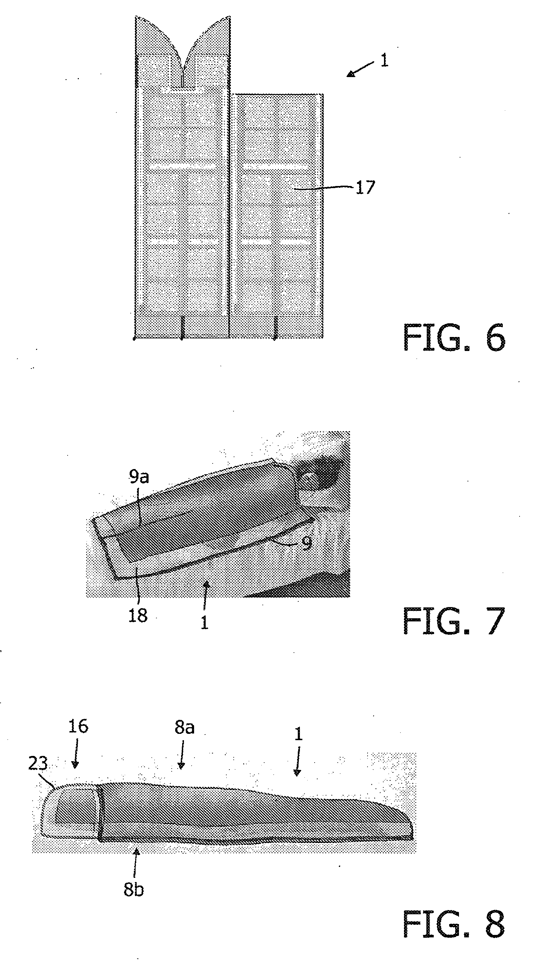

In the cover (not part of the present invention) that is shown in figure 6, a detachable light permeable (translucent to the LED emitting wavelengths) lining 17 is attached on the inside of the cover 1, over the foils 2, so that possible heat that is generated by the LEDs 3 can be decreased. For example, the lining 17 may comprise phase change material. The lining 17 may add to the comfort of the cover 1. The lining 17 may comprise multiple layers that serve various purposes, also the lining 17 can be used instead of, or as a part of above mentioned sheet 8. For example, a liner 17 made of soft and bulky material may add to the comfort.

The lining 17 may have fluid absorbing properties. The lining 17 may act as a diffusing and/or light scattering layer for electroluminescent point sources 3. The lining 17 can for example be taken off the cover 1 for washing and/or exchanging. The lining 17 may comprise a fabric or textile, or any other polymer or rubbery substance in general. Non-woven' fabric linings 17 comprise fibers that are bonded together, for example by use of resins or mechanical entanglement, as compared to woven fabric linings, where the fibers may be held together by weaving of the yarn and/or twisting of the fibers in the yarn. Suitable fibers include natural textile fibers, such as cotton or wool fibers, regenerated fibers, such as viscose, and synthetic fibers such as polyester, polyamide (nylon) or polyacrylic fibers. To get a more homogeneous light emitting surface, multiple layers may be provided, for example a layer of diffusing foam can be placed in between the flexfoils and the lining 17. The lining 17 may also comprise non-woven or spacer fabrics or foam or any other material that diffuses light and create a homogenous light panel out of an assembly of point sources of light such as LEDs 3.

The lining 17 may be configured to withstand and/or absorb faeces and/or fluids coming from the user. For example, the lining 17 can include a diper or the like and/or has properties to repel and/or absorp moisture and bodily fluids. This lining 17 or a layer thereof can be disposable. This might for example be advantageous with an embodiment of a cover 1 for babies, providing a type of nappy function. The lining 17 can be exchanged and washed and the rest of the cover 1 can be kept relatively clean. Alternatively, the cover 1 itself is configured to withstand, repel and/or absorp faeces and/or fluids and/or can be washed, such that the electric components, for example the electroluminescent sources 3 and/or the electric circuit, will not be damaged. Alternatively, the light panels can be encapsulated individually, collectively or in modules in a lining 17 material or for example in polymeric/elastomeric soft materials for protection and robustness. For this, protective layers may be provided in the cover 1, for example, rubbery or polymer materials. Of course multiple linings 17 can be provided for one cover 1.

The use of a cover 1 can for example be as follows. A patient is placed on the cover 1 on the side of the LEDs 3, preferably on the sweat absorbing lining 17, that touches the naked skin. The LEDs 3 expose the body of the patient preferably with blue light. Preferably, the LEDs are configure to emit at a high-intensity narrow band blue light with a wavelength in the 450-470 nm spectrum, centered around a 450 nm peak and no light emission in the UV wavelength area. As shown in figure 2, the foil 2 contain holes, so that body heat and moisture can exit.

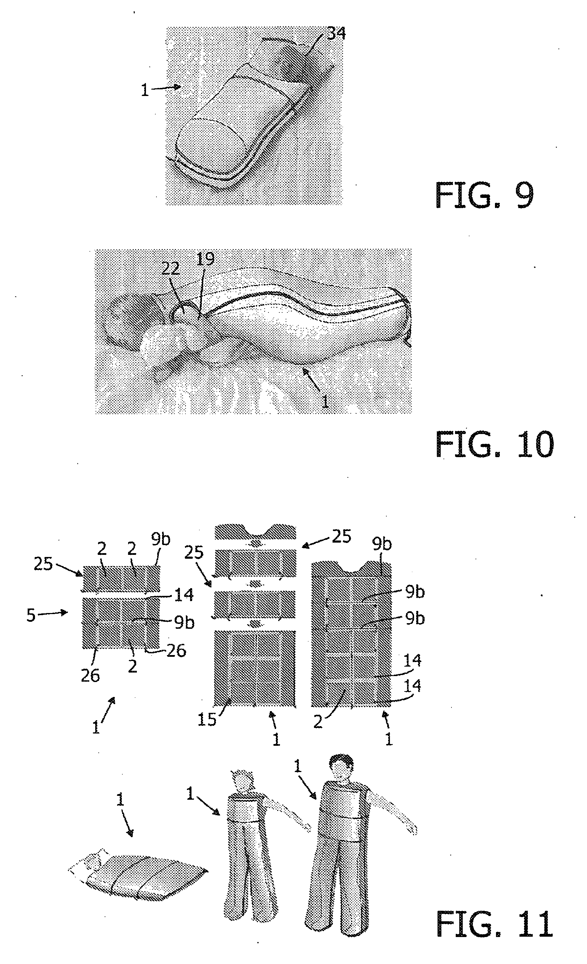

When the patient is placed on the cover 1, the cover may be closed by means of joining means 9. The joining means 9 may comprise zippers 9, velcro, buttons and/or the cover 1 may be attached directly to the patient. When the joining means 9 are joined, a sleeping bag may be formed as shown in f1 figure 8 and 9. The cover 1 may be provided with a pillow 34, which may also comprise electroluminescent sources 3.

Different covers have different sizes. A cover for babies is shaped for babies to one year old (figure 9), other covers are shaped as an infant's sleeping bag for 1 to 4 year old children and for example have removable cotton sleeves 19 (figure 10). In an embodiment a sleeping bag can be resized to three sizes for different treatments, different and/or growing patients, for example for the following age groups: 7 to 12 year old children, 12 to 18 year olds and adult users (figure 11).

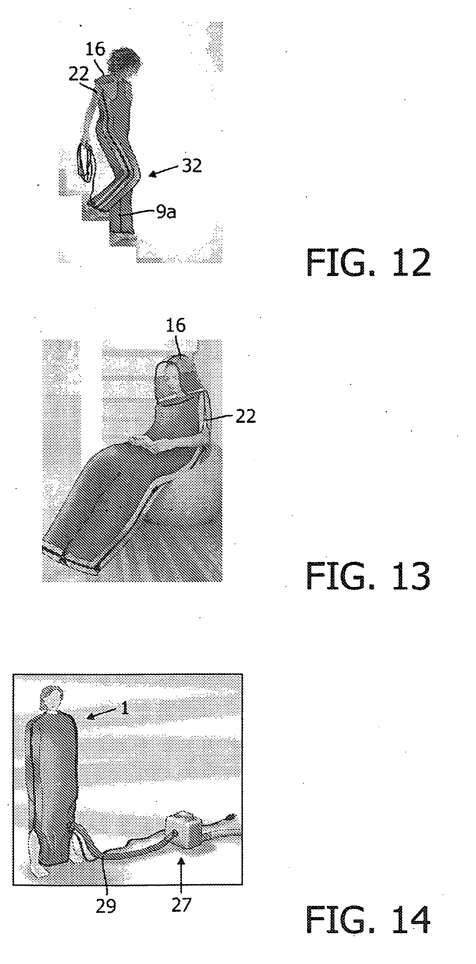

The sleeping bags 1 can consist of two separate sheets 8a, 8b, each covered on one side with foils 2 (see figure 5). For example, zippers 9 are provided on the sides of the cover 1 and by zipping the sheets 8a, 8b a sleeping bag can be created. In particular embodiments, the upper part 20 of the sides have 'pushbuttons' 21 and by leaving these buttons 21 open, arm openings 22 are created (figure 12, 13). In the middle of both sheets 8 a zipper 9a is placed, to create pants 32, providing for a hybrid function for the cover 1. Preferably, no LEDs 3 are placed at knee and hip height, so that the user can comfortably move in the suit.

One of said sheets 8a is provided with flaps 16 containing two extra foils 2. In an embodiment, the flaps 16 are used as shoulder fasteners to expose the shoulders when the cover 1 is used as a suit, as shown in figure 12, and it can be used as a hood when it is used as a sleeping bag or suit, to enlighten the face, as shown in figure 8 and 13. When used as a hood while sleeping, two arcs 23 are provided, so that the hood doesn't touch the face, but is hanging on a distance above the head. This is shown in figure 8. The backing material of the hood is for example made of a net type material to create an open structure and air can flow through. The backing material could for example also comprise a porous material.

Preferably, at the bottom part of the cover 1 extra material 24 is attached that can be pulled up or down the leg. This may comprise elastic material to be able to clamp a part of the cover 1 against the leg.

According to the invention the cover 1 is constructed by joining modular cover parts 25 with foils 2 to each other, as can be seen from figure 11. These building blocks are for example provided with two foils 2 placed side to side. In the shown embodiment, joining means 9 comprise zippers 9b on the edges with which the modular cover parts 25 can be connected to the cover or may build to form a cover 1. The LEDs may for example be connected by plugging small connectors 26. In another embodiment (not shown), the LEDSs 3 automatically connect to connectors 26 by joining the joining means 9b. Another embodiment is provided with conductive zippers 9b or Velcro, such that modular elements 25 connect to the electric circuit by joining the velcro. Other joining means 9b may of course also be conductive and applied for like embodiments. The embodiment provided with the modular parts 25 is for example advantageous when the user is a Crigler-Najjar patient. By means of the variable size, the patient can use the cover 1 for longer periods, for example several years and adjust the cover 1 for his comfort. The cover 1 can be enlarged by attaching a modular part 25 when the cover 1 becomes too small. In an embodiment of the cover 1, between the sheets 8a, 8b two extra modular cover parts, with or without foils 2, can be zipped to enlarge the size of the cover 1, to provide extra space for body movement during sleep.

Alternatively, the cover 1 can be of variable size by means of extra backing material, in which case the upper part of the cover 1 can be folded and, for example, hidden behind a zipper, for example hidden in a zipped pocket. When the user grows and the cover 1 becomes relatively too small, the extra material can be revealed and foils 2 can be placed inside. The extra material already contains the electronic connectors 26 and foil connectors 14 to connect the foil 2 to the system.

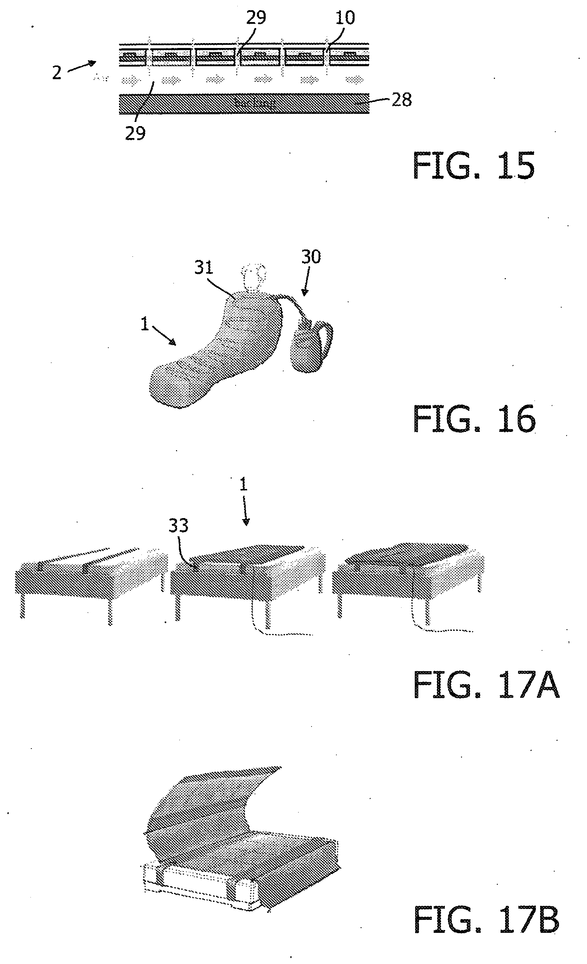

The cover 1 may be provided with a cooling system, for example when the cover 1 and/or the LEDs dissipate a relative large amount of heat. The cooling system for example comprises an airconditioning apparatus (airco) 27 that is connected to the suit and blows cold air 29 between a backing material 28 and the foils 2, as shown in figure 14 and 15. Air may also blow through the openings 10 in the foil 2 onto the body.

Another cover is provided with a liquid cooling system 30, for example as known from military suits. An example of such a cover is shown in figure 16. Cool water is pumped through tube frames 31 in the cover 1 to cool the electronics and/or body. To prevent the risk of overheating, the cover 1 may comprise a built-in sensor that measures the temperature and for example decreases the light intensity when the temperature exceeds a certain level, until the desired temperature is reached again.

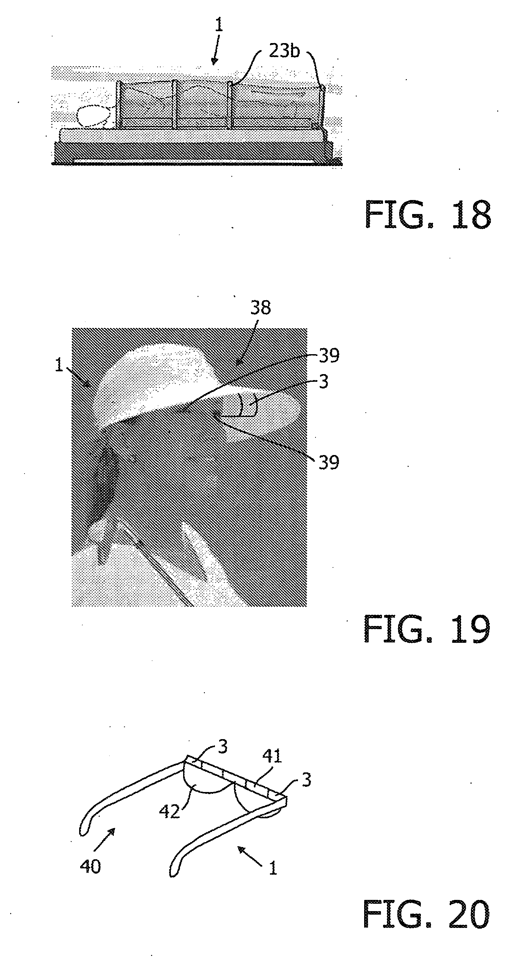

Cooling may also be provided passively by providing the cover 1 with a frame 23b of tubes, for example shaped as hoops or arcs, in the cover, so that an air layer is created between at least a part of the body and the cover 1, as shown in figure 18.

To prevent wrinkling and folding of the foils 2 because of the moving an embodiment of the cover 1 comprises fixation means, for example bands 33, that are for example fixed to one of the sheets 8 and can be placed for example around body support, such as a mattress. Such a configuration is shown in figure 17A and B. The bands 33 pull the sheet 8 tight. The user can now lie on the sheet 8 without the risk of folding it during sleep.

The cover 1, or at least the inside of the cover 1, may be provided with reflecting material so that the light of the LEDs 3 is reflected and possibly more light reaches the part of the body. Light that is transmitted to the back of the LEDs 3, may be reflected back to the front side so that it reaches the body. This will improve the light output and improve the efficiency of the cover 1.

The cover 1 may comprise other electroluminescent sources 3 than the foil 2 with LEDs 3. For example OLEDs 3 and/or LEDs 3 attached to conductive and/or non-conductive yarns 11, 12 may be provided within the scope of the invention. Other LEDs 3 and/or other ways of attaching LEDs 3 and/or other electroluminescent sources to a substrate 2 are obviously possible within the scope of the invention.

The cover may be provided with at least one opening. Next to arm openings 22, openings may be provided for the head, shoes, eyes, ears and/or mouth. Openings may also be provided to pass through food, urine and/or faeces. The openings may be reopenable and/or reclosable.

The control means 5 may receive data from sensors. Sensors and/or other measurement devices are provided to provide data for the control means 5, which will send a signal to the adjustment, if necessary. For example, the status of the illness can be measured by measuring the colour of the skin. Also moisture conditions and/or temperature conditions can be measured with known devices. Another example is measuring the colour of the light, i.e. the wavelength, to check what wavelength is being emitted. In this way, the system, i.e. control means 5, can self-adjust in time, for example according to a pre-set schedule. Wavelength settings and/or intensity settings can be adjusted as well as the temperature and/or moisture conditions of the cover 1.

Blue light in a wavelength spectrum of approximately 440 to 480nm, for example approximately 450nm can be useful for revitalizing a person through the eyes. Therefore, a cover 1 may comprise a hat, cap and/or visor 38, provided with electroluminescent sources 3, for example (O)LEDs 3. The blue light is emitted to the area 39 around the eyes, as can be seen from figure 19. Preferably, the blue light is emitted to the area around the bottom eyelids, from above the forehead. Therefore, preferably a revitalising head cover 1 is provided with LEDs in the cap portion 39. Of course the light of the electroluminescent sources 3 can also be reflected to the area of the eyes 39. Another cover has the shape of glasses 40, where the LEDs can for example be provided in the portion 41 above the glasses 42, as can be seen from figure 20. The revitalising head covers 1 may be provided with transparent electroluminescent sources 3, for example OLEDs 3, such that the electroluminescent sources are attached to a transparent surface, for example in the glasses 42 and/or a transparent visor. These types of revitalising head covers 1 can be revitalising, for example in tiring moments, for example when working at night, for example truck driving at night or other night shift work. There is little loss of comfort. The LEDs can be integrated with the fabric, for example such as explained above; for example by means of a foil 2 and/or integration of LEDs 3 with conductive and non-conductive yarns 11, 12. Flexible substrates and/or flexible electroluminescent sources 3 can be integrated with the fabric of the revitalising head cover 1. Of course OLEDs could be attached to the head cover as well. Different covers involve power supplies such as batteries, fuel cells, etc. Control means could be integrated as well. Attachment means for the electroluminescent sources for example include glue.

As mentioned above, phototherapy can be applied for wound healing. For example, wounds on women's breast, particularly around the nipples during breastfeeding, can also be treated by phototherapy. If these types of breast wounds are not treated in time, infections might occur and/or breastfeeding has to be interrupted. Since breastfeeding may take place for example every three or four hours and each session may take for example up to an hour, there might be insufficient time for the wound(s) to heal. Although for example for pain relief certain creams are sometimes used, those generally do not accelerate breast wound healing.

By applying a cover 1 for emitting light at certain wavelengths to women's breasts, the healing of the wounds, especially near the nipples, can be accelerated and/or wounds and/or infections may be prevented. Therefore it is advantageous when a cover 1 can be worn near the breasts, or at least near the nipples. Furthermore, a cover 1 for breast wound healing may be used to prevent wounds or for cosmetic purposes.

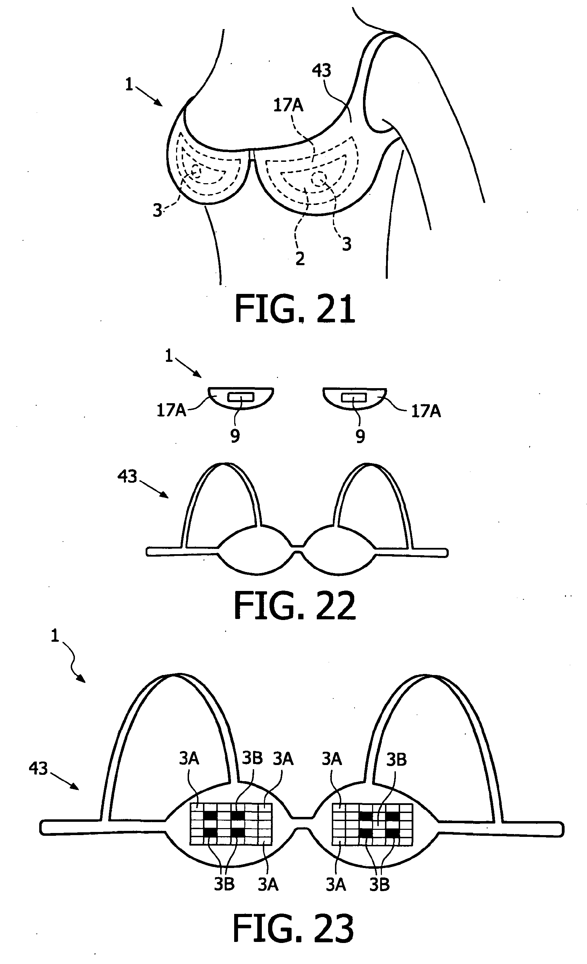

For example, a cover 1 may be at least partly preshaped in the form of a nipple, possibly including at least part of the areola mammae and/or further breast. Also, the cover 1 for breast wound healing can be flexible, wherein it is configured to at least partly follow the curves of the female breast. The cover 1 may be constructed like a bra 43 or a replaceable lining 17A for a bra 43. In figure 21 a bra 43 is shown, which is provided with elements 2, 32, 17A that are indicated with dashed lines. This cover has advantageously combined electroluminescent sources like LEDs 3 with bras 43 so that a cover 1 functions as a phototherapeutic bra 43. The lining 17A may be applied, among others, for comfort, light scattering and for example absorption of leaking fluids from the breast. The electroluminescent sources may comprise LEDs 3 that emit light in the red and/or infrared region, which is advantageous for wound healing. With the aid of control means 5, the wavelength and/or intensity of at least a part of the LEDs can be changed to apply a more optimised treatment. The bra 43 for example comprises foils 2, knitted, woven and/or non-woven textiles 2 and/or conductive and non-conductive yarns 11, 12 to which the LEDs 3 are attached, as already described above. The foil 2 with LEDs 3 can for example be attached to or laid in the inside of the bra 43, wherein for example a lining 17A or another type of layer acts as a diffusing layer. The cover 1 for breast wound healing can be put in a compartment that is provided in a bra 43. In these and other ways, a phototherapeutic bra 43 can be conveniently worn like a conventional bra. Of course, the cover 1 doesn't need to be in direct contact with skin and one or more layers can be put in between the cover 1 and the skin.

The lining 17A may include LEDs 3 and/or a light scattering and/or diffusing layer, and can be placed in the bra 43. The lining 17A may be fixedly integrated with the bra 43 or can be placed in a conventional bra 43 in a replaceable way, as shown in figure 22. For this, the lining 17A may comprise joining means 9, such as for example comprising Velcro-like material, buttons, etc. The cover 1 may comprise a nursing pad or another pad, to be put against a breast. Such a nursing pad may prevent and/or absorb leakage and apply phototherapy by having LEDs 3 applied. The cover 1 for women's breast may be integrated or joinable with T-shirts, tops, patches, etc. To enhance the therapy, phototherapy can be applied in combination with creams and/or agents.

With the aid of sensors and control means 5, as mentioned above, the bra 43 can self adjusts its therapy according to the size and/or state of the wound(s) in time. For this, software and/or predetermined data are used so that the phototherapy can be performed according to measurements and predetermined data. For example, the light intensity of at least a part of the LEDs 3 can be adjusted. Sensors can be used that measure temperature, colour and/or emittance of the skin. The LEDs 3 may be configured to be light sources 3A as well as sensors 3B, as shown in figure 23. In an embodiment, the reflectivity of the skin is measured with the LEDs 3B that sense light that is emitted by the LEDs 3A and reflected by the skin. From this reflectivity information, the state of specific parts of the body, for example the nipples can be obtained by comparing with predetermined data and/or changes of the nipples in time, for example hours or days. The lining 17A and/or LEDs 3 may be shaped to fit around a nipple and/or the area around the nipple.

Many variations are possible within the framework of the invention as outlined by the claims. A cover 1 may for example configured for any type of phototherapy, not specifically described in the above text.

For instance, although in relation to wound healing a bra is discussed, other body covers discussed in the description can be used for wound healing purposes too.

Furthermore the body cover, used for wound healing can also be used for pain relief, e.g. for the treatment of muscle pain or joint pain.

## Claims

1. Body cover (1) for covering at least part of a human body, wherein the cover is configured to at least partly surround at least a part of the human body and/or a body support, wherein the body cover (1) comprises multiple modular body cover parts (25) that are joined together to form the body cover (1), wherein a plurality of the multiple body cover parts (25) comprise at least one electroluminescent source (3) coupled to a substrate (2), for illuminating at least a part of the human body; and wherein the substrate (2) comprises a flexible and foldable foil, **characterised in that** the multiple modular body cover parts are detachably joined together.

2. Body cover according to claim 1, wherein the at least one electroluminescent source (3) is configured to emit blue light between a wavelength of 440nm and 480nm, preferably between 445nm and 470nm, more preferably approximately 450 nm.

3. Body cover according to any one of the preceding claims, wherein the cover comprises an element (16) configured to cover at least a part of a head to illuminate at least one eye.

4. Body cover according to any one of the preceding claims, having a thermal conditions and/or moisture conditions regulating configuration.

5. Body cover according to any one of the preceding claims, comprising control means (5) provided with wavelength settings and/or intensity settings adjusting means, for the at least one electroluminescent source (3), said means preferably comprising local settings adjustment means.

6. Body cover according to claim 4 or 5, wherein control means (5) are provided, comprising at least a processing circuit, wherein the processing circuit is configured to adjust and/or monitor at least one of said conditions and/or settings, preferably by measuring said at least one of said settings and/or conditions, comparing at least one measured value to at least one pre-set value and adjusting/keeping said at least one of said settings and/or conditions in correspondence with the pre-set value.

7. Body cover according to any one of the claims 5 or 6, wherein said modular cover part(s) (25) are configured to connect to the control means (5).

8. Body cover according to claim 7, wherein joining means (9b) are configured for connecting said modular cover parts(s) (25) by joining the joining means (9b).

9. Body cover according to any one of the preceding claims, wherein openings (10) are provided in the at least one substrate and/or in the at least one electroluminescent source and/or between multiple electroluminescent sources (3).

10. Body cover according to any one of the preceding claims, wherein the at least one substrate (2) comprises at least one sheet (8) of foil, wherein the foil comprises knitted textile and/or polymers, rubbery materials, spacer fabrics, and wherein the at least one electroluminescent source (3) is attached to the at least one sheet (8).

11. Body cover according to claim 10, wherein said sheet (8) comprises at least one partly conductive yarn (11, 12), wherein the at least one electroluminescent source (3) is attached to and/or between at least partly conductive yarns (11, 12).

12. Body cover according to any one of the preceding claims, wherein the cover (1) comprises a frame (23; 23b) for keeping the cover in a substantially pre-shaped condition and/or for allowing temperature regulating means (27, 29; 30, 31) to pass through.

13. Body cover as claimed in claim 1, comprising sensor(s) for measuring at least one of body colour, temperature, humidity, wavelength and/or intensity of the light and means (5) for comparing at least one of the measured values with at least one pre-set value and adjusting the wavelength and/or intensity of the at least one electroluminescent source (3) according to the pre-set.

14. Body cover as claimed in claim 1, comprising means (6) for adjusting the wavelength of at least one electroluminescent source locally, whereas another (part of) the electroluminescent source (3) emits at a substantially different wavelength then the adjusted wavelength value, or doesn't emit.

15. Body cover according to any one of any one of the preceding the claims, wherein the cover (1) is a breast cover (43) for covering at least part of a women's breast, and wherein it is at least partially configured to follow the curves of women's breasts, preferably to function as a bra.

16. Body cover according to claim 15, comprising a light diffusing layer (17A).

## Patentansprüche

1. Körperabdeckung (1) zum Abdecken mindestens eines Teils eines menschlichen Körpers, wobei die Abdeckung konfiguriert ist, um mindestens einen Teil des menschlichen Körpers und/oder einer Körperauflage mindestens teilweise zu umgeben, wobei die Körperabdeckung (1) mehrere modulare Körperabdeckungsteile (25) umfasst, die zusammengefügt sind, um die Körperabdeckung (1) zu bilden, wobei eine Vielzahl von mehreren Körperabdeckungsteilen (25) mindestens eine mit einem Substrat (2) gekoppelte elektrolumineszente Quelle (3) umfassen, um mindestens einen Teil des menschlichen Körpers zu bestrahlen; und wobei das Substrat (2) eine flexible und faltbare Folie umfasst, **dadurch gekennzeichnet, dass** die mehreren modularen Körperabdeckungsteile lösbar zusammengefügt sind.

2. Körperabdeckung nach Anspruch 1, wobei die mindestens eine elektrolumineszente Quelle (3) konfiguriert ist, um blaues Licht mit einer Wellenlänge zwischen 440 nm und 480 nm, vorzugsweise zwischen 445 nm und 470 nm, und noch stärker zu bevorzugen mit einer Wellenlänge von ca. 450 nm, zu emittieren.

3. Körperabdeckung nach einem der vorhergehenden Ansprüche, wobei die Abdeckung ein Element (16) umfasst, das konfiguriert ist, um mindestens einen Teil eines Kopfes abzudecken, um mindestens ein Auge zu bestrahlen.

4. Körperabdeckung nach einem der vorhergehenden Ansprüche, mit einer die thermischen Bedingungen und/oder die Feuchtigkeitsbedingungen regulierenden Konfiguration.

5. Körperabdeckung nach einem der vorhergehenden Ansprüche, mit Steuermitteln (5), die mit Mitteln zur Justierung der Wellenlängeneinstellungen und/oder der Intensitätseinstellungen für die mindestens eine elektrolumineszente Quelle (3) ausgestattet sind, wobei die genannten Mittel vorzugsweise lokale Einstellungsjustierungsmittel umfassen.

6. Körperabdeckung nach Anspruch 4 oder 5, wobei Steuermittel (5) vorgesehen sind, die mindestens eine Verarbeitungsschaltung umfassen, wobei die Verarbeitungsschaltung konfiguriert ist, um mindestens eine der genannten Bedingungen und/oder Einstellungen zu justieren und/oder zu überwachen, vorzugsweise durch Messen der genannten mindestens einen der genannten Einstellungen und/oder Bedingungen, Vergleichen des mindestens einen gemessenen Wertes mit mindestens einem vorgegebenen Wert und Justieren/Halten der genannten mindestens einen der genannten Einstellungen und/oder Bedingungen in Übereinstimmung mit dem vorgegebenen Wert.

7. Körperabdeckung nach einem der Ansprüche 5 oder 6, wobei die genannten modularen Abdeckungsteile (35) konfiguriert sind, um die Steuermittel (5) zu verbinden.

8. Körperabdeckung nach Anspruch 7, wobei Zusammenfügungsmittel (9b) konfiguriert sind, um die genannten modularen Abdeckungsteile (25) durch Zusammenfügen der Zusammenfügungsmittel (9b) zu verbinden.

9. Körperabdeckung nach einem der vorhergehenden Ansprüche, wobei Öffnungen (10) in dem mindestens einen Substrat und/oder in der mindestens einen elektroluminszenten Quelle und/oder zwischen mehreren elektrolumineszenten Quellen (3) vorgesehen sind.

10. Körperabdeckung nach einem der vorhergehenden Ansprüche, wobei das mindestens eine Substrat (2) mindestens ein Folienblatt (8) umfasst, wobei die Folie gestricktes Textil und/oder Polymere, gummiartige Materialien, Abstandsstoff umfasst, und wobei die mindestens eine elektrolumineszente Quelle (3) an dem mindestens einen Blatt (8) angebracht ist.

11. Körperabdeckung nach Anspruch 10, wobei das genannte Blatt (8) mindestens ein teilweise leitendes Garn (11, 12) umfasst, wobei die mindestens eine elektroluminseszente Quelle (3) an und/oder zwischen mindestens teilweise leitenden Garnen (11, 12) angebracht ist.

12. Körperabdeckung nach einem der vorhergehenden Ansprüche, wobei die Abdeckung (1) einen Rahmen (23, 23b) umfasst, um die Abdeckung in einem im Wesentlichen vorgeformten Zustand zu halten und/oder um den Temperaturregulierungsmitteln (27, 29; 30, 31) den Durchgang zu erlauben.

13. Körperabdeckung nach Anspruch 1, mit Sensor(en) zum Messen von mindestens entweder der Körperfarbe, der Temperatur, der Feuchtigkeit, der Wellenlänge und/oder der Intensität des Lichts, und Mitteln (5) zum Vergleichen von mindestens einem der gemessenen Werte mit mindestens einem vorgegebenen Wert und Justieren der Wellenlänge und/oder Intensität der mindestens einen elektrolumineszenten Quelle (3) entsprechend der Vorgabe.

14. Körperabdeckung nach Anspruch 1, mit Mitteln (6) zum lokalen Justieren der Wellenlänge der mindestens einen elektrolumineszenten Quelle, während eine andere (bzw. ein anderer Teil der) elektrolumineszente(n) Quelle (3) bei einer wesentlich anderen Wellenlänge als dem justierten Wellenlängenwert emittiert, oder nicht emittiert.

15. Körperabdeckung nach einem der vorhergehenden Ansprüche, wobei die Abdeckung (1) eine Brustabdeckung (43) zum Abdecken mindestens eines Teils einer weiblichen Brust ist, und wobei sie zumindest teilweise konfiguriert ist, um den Kurven der weiblichen Brust zu folgen, vorzugsweise um als Büstenhalter zu fungieren.

16. Körperabdeckung nach Anspruch 15, mit einer lichtstreuenden Schicht (17A).

## Revendications

1. Enveloppe corporelle (1) pour recouvrir au moins une partie d'un corps humain, dans laquelle l'enveloppe est configurée pour au moins partiellement entourer au moins une partie du corps humain et/ou un support corporel, dans laquelle l'enveloppe corporelle (1) comprend plusieurs parties d'enveloppe corporelle modulaires (25) qui sont jointes l'une à l'autre pour former l'enveloppe corporelle (1), dans laquelle une pluralité des plusieurs parties d'enveloppe corporelle (25) comprennent au moins une source électroluminescente (3) couplée à un substrat (2), pour éclairer au moins une partie du corps humain ; et dans laquelle le substrat (2) comprend un film souple et pliable, **caractérisée en ce que** les plusieurs parties d'enveloppe corporelle modulaires sont jointes l'une à l'autre de manière détachable.

2. Enveloppe corporelle selon la revendication 1, dans laquelle l'au moins une source électroluminescente (3) est configurée pour émettre de la lumière bleue entre une longueur d'onde de 440 nm et 480 nm, de préférence entre 445 nm et 470 nm, avec plus de préférence environ 450 nm.

3. Enveloppe corporelle selon l'une quelconque des revendications précédentes, dans laquelle l'enveloppe comprend un élément (16) configuré pour recouvrir au moins une partie d'une tête pour éclairer au moins un oeil.

4. Enveloppe corporelle selon l'une quelconque des revendications précédentes, comportant une configuration de régulation des conditions thermiques et/ou des conditions d'humidité.

5. Enveloppe corporelle selon l'une quelconque des revendications précédentes, comprenant un moyen de commande (5) pourvu d'un moyen de réglage de paramètres de longueur d'onde et/ou de paramètres d'intensité, pour l'au moins une source électroluminescente (3), ledit moyen comprenant de préférence un moyen de réglage de paramètre local.

6. Enveloppe corporelle selon la revendication 4 ou 5, dans laquelle le moyen de commande (5) est pourvu d'au moins un circuit de traitement, dans laquelle le circuit de traitement est configuré pour régler et/ou surveiller au moins l'un desdits paramètres et/ou conditions, de préférence en mesurant ledit au moins l'un desdits paramètres et/ou conditions, en comparant au moins une valeur mesurée à au moins une valeur prédéfinie et en réglant/maintenant ledit au moins l'un desdits paramètres et/ou conditions en correspondance avec la valeur prédéfinie.

7. Enveloppe corporelle selon l'une quelconque des revendications 5 et 6, dans laquelle lesdites parties d'enveloppe modulaires (25) sont configurées pour être reliées au moyen de commande (5).

8. Enveloppe corporelle selon la revendication 7, dans laquelle un moyen de jonction (9b) est configuré pour relier lesdites parties d'enveloppe modulaires (25) en les joignant par le moyen de jonction (9b).

9. Enveloppe corporelle selon l'une quelconque des revendications précédentes, dans laquelle des ouvertures (10) sont fournies dans l'au moins un substrat et/ou dans l'au moins une source électroluminescente et/ou entre plusieurs sources électro-luminescentes (3).

10. Enveloppe corporelle selon l'une quelconque des revendications précédentes, dans laquelle l'au moins un substrat (2) comprend au moins une feuille (8) de film, dans laquelle le film comprend du textile tricoté et/ou des polymères, des matières caoutchouteuses, des tissus structurels, et dans laquelle l'au moins une source électroluminescente (3) est attachée à l'au moins une feuille (8).

11. Enveloppe corporelle selon la revendication 10, dans laquelle ladite feuille (8) comprend au moins un fil partiellement conducteur (11, 12), dans laquelle l'au moins une source électroluminescente (3) est attachée entre des fils au moins partiellement conducteurs (11, 12) et/ou à ceux-ci.

12. Enveloppe corporelle selon l'une quelconque des revendications précédentes, dans laquelle l'enveloppe (1) comprend un cadre (23 ; 23b) pour maintenir l'enveloppe dans une condition sensiblement préformée et/ou pour permettre le passage d'un moyen de régulation de température (27, 29 ; 30, 31) à travers celui-ci.

13. Enveloppe corporelle selon la revendication 1, comprenant un ou plusieurs capteurs pour mesurer au moins l'une de la couleur du corps, la température, l'humidité, la longueur d'onde et/ou l'intensité de la lumière et un moyen (5) pour comparer au moins l'une des valeurs mesurées à au moins une valeur prédéfinie et régler la longueur d'onde et/ou l'intensité de l'au moins une source électroluminescente (3) en fonction de la valeur prédéfinie.

14. Enveloppe corporelle selon la revendication 1, comprenant un moyen (6) pour régler la longueur d'onde d'au moins une source électroluminescente localement, alors qu'une autre partie de la source électroluminescente (3) émet à une longueur d'onde sensiblement différente de la valeur de longueur d'onde réglée ou n'émet pas du tout.

15. Enveloppe corporelle selon l'une quelconque des revendications précédentes, dans laquelle l'enveloppe (1) est une enveloppe de poitrine (43) pour recouvrir au moins une partie de la poitrine d'une femme, et dans laquelle elle est au moins partiellement configurée pour suivre les courbes de la poitrine d'une femme, de préférence pour servir de soutien-gorge.

16. Enveloppe corporelle selon la revendication 15, comprenant une couche de diffusion de lumière (17A).
